(19) Europäisches Patentamt
European Patent Office
Office européen des brevets

(11) Veröffentlichungsnummer : **0 337 199 B1**

(12) # EUROPÄISCHE PATENTSCHRIFT

(45) Veröffentlichungstag der Patentschrift : **29.01.92 Patentblatt 92/05**

(51) Int. Cl.$^5$ : **C07D 249/08,** C07D 233/56, C07D 409/06, C07D 405/06, C07D 413/06, A01N 43/653, A01N 43/50, A01N 43/80

(21) Anmeldenummer : **89105586.5**

(22) Anmeldetag : **30.03.89**

(54) **Azolylalkene und diese enthaltende Fungizide.**

Die Akte enthält technische Angaben, die nach dem Eingang der Anmeldung eingereicht wurden und die nicht in dieser Patentschrift enthalten sind.

(30) Priorität : **09.04.88 DE 3811916**

(43) Veröffentlichungstag der Anmeldung : **18.10.89 Patentblatt 89/42**

(45) Bekanntmachung des Hinweises auf die Patenterteilung : **29.01.92 Patentblatt 92/05**

(84) Benannte Vertragsstaaten : **AT BE CH DE ES FR GB GR IT LI NL SE**

(56) Entgegenhaltungen :
**EP-A- 0 008 651**
**EP-A- 0 094 167**
**EP-A- 0 132 730**
**EP-A- 0 299 683**
**DE-A- 2 652 313**

(73) Patentinhaber : **BASF Aktiengesellschaft**
**Carl-Bosch-Strasse 38**
**W-6700 Ludwigshafen (DE)**

(72) Erfinder : **Karbach, Stefan, Dr.**
**Grundwiesenweg 44**
**W-6730 Neustadt (DE)**
Erfinder : **Seele, Rainer, Dr.**
**Leiblstrasse 3**
**W-6701 Fussgoenheim (DE)**
Erfinder : **Kober, Reiner, Dr.**
**Im Schlittweg 20**
**W-6701 Fussgoenheim (DE)**
Erfinder : **Ammermann, Eberhard, Dr.**
**Sachsenstrasse 3**
**W-6700 Ludwigshafen (DE)**
Erfinder : **Lorenz, Gisela, Dr.**
**Erlenweg 13**
**W-6730 Neustadt (DE)**

**EP 0 337 199 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Azolylalkene, Verfahren zu ihrer Herstellung und diese enthaltende Fungizide.

Es ist bekannt, 1-(1,2,4-Triazol-1-yl)-2-(2,4-dichlorphenyl)-pentan als Fungizid zu verwenden (C.A. No. 66246-88-6). Seine fungizide Wirkung ist jedoch unbefriedigend. Es ist ferner bekannt, Triazolylpropenderivate als Fungizide zu verwenden (DE-A-2652313, EP-A-132730, EP-A-94167). Die ältere Patentanmeldung EP-A-299683 betrifft fungizide Azolylalkene.

Es wurde nun gefunden, daß Azolylalkene der allgemeinen formel I

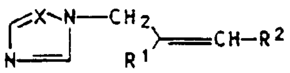

I

in welcher $R^1$ Fluorphenyl,
$R^2$ Naphthyl, einen 5- oder 6-gliedrigen Heterocyclus, Biphenyl oder Phenyl, wobei diese Reste gegebenenfalls einfach bis dreifach durch Halogen, Nitro, Amino, Phenoxy, Alkyl, Alkoxy oder Halogenalkyl mit jeweils 1 bis 4 Kohlenstoffatomen substituiert sind, und
X CH oder N bedeuten, sowie deren für Pflanzen verträgliche Säureadditionssalze oder Metallkomplexe, außer den Verbindungen, in denen $R^1$ 4-Fluorphenyl oder 2,4-Difluorphenyl und gleichzeitig $R^2$ 4-Fluorphenyl, 4-Chlorphenyl, 4-Methoxyphenyl, 4-Trifluormethylphenyl oder Pyridyl-4-bedeuten, eine bessere fungizide Wirkung, insbesondere gegen Getreidekrankheiten, besitzen als die bekannten Azolylalkane.
$R^1$ bedeutet beispielsweise : einfach bis fünffach, insbesondere einfach bis zweifach, durch Fluor substituiertes Phenyl, 2-Fluorphenyl, 3-Fluorphenyl, 4-Fluorphenyl, 3,4-Difluorphenyl, 2,4-Difluorphenyl, 2,6-Difluorphenyl, 2,4,6-Trifluorphenyl, Pentafluorphenyl ;
$R^2$ bedeutet beispielsweise : 1-Naphthyl, 2-Naphthyl, p-Biphenyl, Phenyl, Halogenphenyl, 2-Chlorphenyl, 2-Fluorphenyl, 2-Bromphenyl 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, Alkoxyphenyl 2-Methoxyphenyl, 3-Methoxyphenyl, 4-Methoxyphenyl, 2,4-Dimethoxyphenyl, Alkylphenyl, 4-Ethylphenyl, 4-Isopropylphenyl, 4-tert.-Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Aminophenyl, 2-Trifluormethylphenyl, 3-Trifluormethylphenyl, 4-Trifluormethylphenyl, den Rest eines 5- oder 6-gliedrigen Heterocyclus, der 1 oder 2 Sauerstoffatome, Stickstoff- oder Schwefelatome enthält und gesättigt oder ungesättigt ist, z.B. Dioxan, Thiophen, Furan, Pyran, Tetrahydropyran, Pyridin, Isoxazol, Methyl-isoxazol, beispielsweise 1,3-Dioxan-2-yl, 1,4-Dioxan-2-yl, 2-Thienyl, 3-Thienyl, 2-Furanyl, 3-Furanyl, Tetrahydropyranyl, 3-Pyridyl, 5-(3-Methylisoxazolyl).

Säureadditionssalze der Wirkstoffe I sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß das Anion i.a. beliebig gewählt werden kann. Die Wirkstoffsalze werden hergestellt durch Umsetzung der Azolylalkene (I) mit den Säuren.

Metallkomplexe der Wirkstoffe I oder ihrer Salze können z.B. mit Kupfer, Zink, Zinn, Mangan, Eisen, Kobalt oder Nickel gebildet werden, indem man die Azolylalkene mit entsprechenden Metallsalzen umsetzt, z.B. mit Kupfersulfat, Zinksulfat, Zinkchlorid.

Die Verbindungen der Formel I können z.B. hergestellt werden, indem man eine Verbindung der Formel II

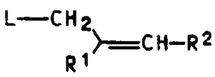

II

in welcher $R^1$, $R^2$ die oben angegebenen Bedeutungen haben und L eine nucleophil substituierbare Abgangsgruppe (Halogen, OH, Tosylat, Mesylat, Triflat) darstellt, mit einer Verbindung der Formel III

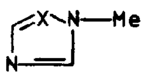

III,

in der Me ein Wasserstoffatom oder ein Metallatom (Na, K) bedeutet und X die oben angegebene Bedeutung

hat, zur Umsetzung bringt.

Die Reaktion erfolgt — falls Me ein Wasserstoffatom bedeutet — gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrollidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium-, Kalium- oder Cäsiumcarbonat oder Natrium-, Kalium- oder Cäsiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumjodid oder Kaliumjodid, quartäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 150°C, drucklos oder unter Druck, kontinuierlich oder diskontinuierlich durchgeführt.

Ist Me ein Metallatom wird die Reaktion gegebenenfalls in Gegenwart eines Lösungs- oder Verdünnungsmittels durchgeführt. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrollidon, ferner Dimethylsulfoxid, Sulfolan oder entsprechende Gemische.

Man arbeitet im allgemeinen zwischen 0 und 100°C, vorzugsweise bei 20 bis 80°C. Bei Anwesenheit eines Lösungsmittels mit niedrigem Siedepunkt wird zweckmäßigerweise bei der Siedetemperatur gearbeitet.

Die Verbindung III stellt man her, indem man z.B. Olefine der Formel IV

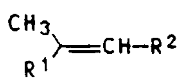

IV

nach bekannten Methoden in Allylposition halogeniert oder oxidiert.

Geeignete Halogenierungsreagenzien sind N-Chlor- und N-Bromsuccinimid in halogenierten Kohlenwasserstoffen wie Tetrachlorkohlenstoff, Trichlorethan, Methylenchlorid oder Chlorbenzol bei Temperaturen zwischen 20 und 100°C. Zur Allyloxidation verwendet man Perester wie Perbenzoesäuretert.-butylester oder Peressigsäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100°C.

Die aus einer Oxidation erhaltenen Alkohole werden mit Methansulfonsäurechlorid, Toluolsulfonsäurechlorid, Benzolsulfonsäurechlorid oder Trifluormethylsulfonylchlorid unter Anwendung einer Hilfsbase in inerten Lösungsmitteln zu den Estern der Verbindungen der Formel III umgesetzt, oder auch durch Einwirkung von Halogenwasserstoffsäuren in die Allylhalogenide überführt.

Die Verbindungen IV lassen sich entsprechend allgemein bekanten Verfahren zur Olefinsynthese (Houben-Weyl-Müller, Methoden der organischen Chemie, Georg Thieme Verlag, Stuttgart, 1972 Bd. V, 1b) herstellen.

Man kann auch substituierte Diphenylpropenale der Formel

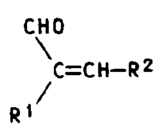

als Ausgangsprodukte verwenden, die zu den Allylalkoholen der Formel

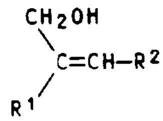

reduziert werden. Die Allylalkohole können mit den oben beschriebenen Umsetzungen in die Azolylalkene überführt werden.

Die Wirkstoffe können als Z- oder E-Isomere auftreten. Die vorliegende Erfindung betrifft beide Formen sowie die Isomerengemische mit unterschiedlichen Isomerenverhältnissen und Fungizide, die eine dieser Formen oder Gemische enthalten.

Die folgenden Beispiele erläutern die Herstellung der Wirkstoffe.

1. Herstellung der Ausgangsstoffe

Vorschrift 1

E/Z-2-(4-Fluorphenyl)-3-(2-chlorphenyl)-propenal

Zu einer Lösung von 35 g 2-Chlorbenzaldehyd in 300 ml Methanol werden 4,2 g Natriumhydroxid in 30 ml Wasser gegeben. Das Reaktionsgemisch wird auf 10°C gekühlt und schnell 36 g 4-Fluorphenylacetaldehyd zugesetzt, wobei die Temperatur in der Lösung auf 30-40°C ansteigt. Nach 10-stündigem Rühren bei 40°C werden die ausgefallenen Kristalle aus der abgekühlten Reaktionslösung abgesaugt.

Vorschrift 2

E/Z-2-(4-Fluorphenyl)-3-(2-chlorphenyl)-allylalkohol

Eine Lösung von 26 g E/Z-2-(4-Fluorphenyl)-3-(2-chlorphenyl)-propenal in 150 ml Isopropanol wird tropfenweise mit Natriumborhydrid-Lösung versetzt, bis chromatographisch kein Startmaterial mehr nachgewiesen werden kann. Die Reaktionsmischung wird auf Eiswasser gegeben und mit Dichlormethan extrahiert. Trocknen und Einengen der organischen Phase ergeben 25 g öliges Endprodukt.

Vorschrift 3

E/Z-2-(4-Fluorphenyl)-3-(2-chlorphenyl)-allylbromid

25 g E/Z-2-(4-Fluorphenyl)-3-(2-chlorphenyl)-allylalkohol werden in 150 ml Methylenchlorid gelöst und in einem langsamen Strom über mehrere Stunden HBr-Gas durchgeleitet. Wenn kein Allylalkohol mehr nachweisbar ist, erfolgt eine wässrige Aufarbeitung mit verdünnter Sodalösung. Trocknung und Abdestillieren der org. Phase ergeben 31 g öliges E/Z-2-(4-Fluorphenyl)-3-(2-chlorphenyl)-allylbromid.

Vorschrift 4

Z-2-(4-Fluorphenyl)-3-(2-chlorphenyl)-allylbromid

E/Z-2-(4-Fluorphenyl)-3-(2-chlorphenyl)-allylbromid wird einige Stunden auf 200°C erhitzt. Hierbei wird das eingesetzte Isomerenverhältnis zu Gunsten des Z-Isomeren verschoben. Die Isolierung des Z-Isomeren erfolgt durch Umkristallisation der erhaltenen Reaktionsmischung aus Isopropanol. Z-2-(4-Fluorphenyl)-3-(2-chlorphenyl)-allylbromid hat einen Schmelzpunkt von 71-73°C.

II. Herstellung der Endprodukte

Beispiel 1

10 g Z-2-(4-Fluorphenyl)-3-(2-chlorphenyl)-allylbromid werden in 70 ml DMF gelöst und 4 g Triazol sowie 6 g Kaliumcarbonat zugefügt. Während des Rührens der Mischung bei Raumtemperatur (20°C) wird durch dünnschichtchromatographische Kontrolle die Abnahme des Gehalts an Allylbromid verfolgt. Die Aufarbeitung

der Reaktionslösung nach beendeter Reaktion (4 Stunden) erfolgt durch Eingießen in Eiswasser und Extraktion mit Methylenchlorid. Es wird Z-1-(1,2,4-Triazol-1-yl)-2-(4-fluorphenyl)-3-(2-chlorphenyl)-allyl (Verbindung Nr. 1) mit einem Schmelzpunkt von 105-107°C isoliert.

Das E-Isomere wird in gleicher Weise durch Umsetzung des chromatographisch gereinigten E-Allylbromids mit Triazol und Kaliumcarbonat in Dimethylformamid erhalten.

Die in der folgenden Tabelle aufgeführten Verbindungen werden nach analoger Vorschrift erhalten :

Tabelle

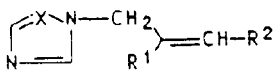

| Nr. | $R^1$ | $R^2$ | Isomer | X | Schmp. |
|---|---|---|---|---|---|
| 1 | 4-Fluorphenyl | 2-Chlorphenyl | Z | N | 107°C |
| 2 | 4-Fluorphenyl | 2-Chlorphenyl | E | N | 63-74°C |
| 3 | 4-Fluorphenyl | 2-Chlorphenyl | Z | CH | 104-106°C |
| 4 | 4-Fluorphenyl | 2-Chlorphenyl | E | CH | 73-81°C |
| 9 | 4-Fluorphenyl | 2,4-Dichlorphenyl | Z | N | |
| 10 | 4-Fluorphenyl | 2,4-Dichlorphenyl | E | N | |
| 11 | 4-Fluorphenyl | 2,4-Dichlorphenyl | Z | CH | |
| 12 | 4-Fluorphenyl | 2,4-Dichlorphenyl | E | CH | |
| 13 | 4-Fluorphenyl | 2-Thienyl | Z | N | 106°C |
| 14 | 4-Fluorphenyl | 2-Thienyl | E | N | |
| 15 | 4-Fluorphenyl | 2-Thienyl | Z | CH | |
| 16 | 4-Fluorphenyl | 2-Thienyl | E | N | |
| 17 | 4-Fluorphenyl | 3-Thienyl | Z | N | |
| 18 | 4-Fluorphenyl | 3-Thienyl | E | N | |
| 19 | 4-Fluorphenyl | 3-Thienyl | Z | CH | |
| 20 | 4-Fluorphenyl | 3-Thienyl | E | CH | |
| 21 | 4-Fluorphenyl | 2-Furanyl | Z | N | 84°C |
| 22 | 4-Fluorphenyl | 2-Furanyl | E | N | |
| 23 | 4-Fluorphenyl | 2-Furanyl | Z | CH | |
| 24 | 4-Fluorphenyl | 2-Furanyl | E | CH | |
| 25 | 4-Fluorphenyl | 3-Furanyl | Z | N | |
| 26 | 4-Fluorphenyl | 3-Furanyl | E | N | |
| 27 | 4-Fluorphenyl | 3-Furanyl | Z | CH | |
| 28 | 4-Fluorphenyl | 3-Furanyl | E | CH | |
| 33 | 4-Fluorphenyl | 5-(3-Methylisoxazolyl) | Z | N | Harz |
| 34 | 4-Fluorphenyl | 5-(3-Methylisoxazolyl) | E | N | |
| 35 | 4-Fluorphenyl | 5-(3-Methylisoxazolyl) | Z | CH | |

| Nr. | $R^1$ | $R^2$ | Isomer | X | Schmp. |
|---|---|---|---|---|---|
| 36 | 4-Fluorphenyl | 5-(3-Methylisoxazolyl) | E | CH | |
| 37 | 2-Fluorphenyl | 2-Chlorphenyl | Z | N | |
| 38 | 2-Fluorphenyl | 2-Chlorphenyl | E | N | |
| 39 | 2-Fluorphenyl | 2-Chlorphenyl | Z | CH | |
| 40 | 2-Fluorphenyl | 2-Chlorphenyl | E | CH | |
| 41 | 2-Fluorphenyl | 4-Chlorphenyl | Z | N | |
| 42 | 2-Fluorphenyl | 4-Chlorphenyl | E | N | |
| 43 | 2-Fluorphenyl | 4-Chlorphenyl | Z | CH | |
| 44 | 2-Fluorphenyl | 4-Chlorphenyl | E | CH | |
| 45 | 2-Fluorphenyl | 2,4-Dichlorphenyl | Z | N | |
| 46 | 2-Fluorphenyl | 2,4-Dichlorphenyl | E | N | |
| 47 | 2-Fluorphenyl | 2,4-Dichlorphenyl | Z | CH | |
| 48 | 2-Fluorphenyl | 2,4-Dichlorphenyl | E | CH | |
| 49 | 2-Fluorphenyl | 2-Thienyl | Z | N | |
| 50 | 2-Fluorphenyl | 2-Thienyl | E | N | |
| 51 | 2-Fluorphenyl | 2-Thienyl | Z | CH | |
| 52 | 2-Fluorphenyl | 2-Thienyl | E | N | |
| 53 | 2-Fluorphenyl | 3-Thienyl | Z | N | |
| 54 | 2-Fluorphenyl | 3-Thienyl | E | N | |
| 55 | 2-Fluorphenyl | 3-Thienyl | Z | CH | |
| 56 | 2-Fluorphenyl | 3-Thienyl | E | CH | |
| 57 | 2-Fluorphenyl | 2-Furanyl | Z | N | |
| 58 | 2-Fluorphenyl | 2-Furanyl | E | N | |
| 59 | 2-Fluorphenyl | 2-Furanyl | Z | CH | |
| 60 | 2-Fluorphenyl | 2-Furanyl | E | CH | |
| 61 | 2-Fluorphenyl | 3-Furanyl | Z | N | |
| 62 | 2-Fluorphenyl | 3-Furanyl | E | N | |
| 63 | 2-Fluorphenyl | 3-Furanyl | Z | CH | |
| 64 | 2-Fluorphenyl | 3-Furanyl | E | CH | |
| 69 | 2-Fluorphenyl | 5-(3-Methylisoxazolyl) | Z | N | |
| 70 | 2-Fluorphenyl | 5-(3-Methylisoxazolyl) | E | N | |
| 71 | 2-Fluorphenyl | 5-(3-Methylisoxazolyl) | Z | CH | |
| 72 | 2-Fluorphenyl | 5-(3-Methylisoxazolyl) | E | CH | |
| 73 | 3-Fluorphenyl | 2-Chlorphenyl | Z | N | |
| 74 | 3-Fluorphenyl | 2-Chlorphenyl | E | N | |
| 75 | 3-Fluorphenyl | 2-Chlorphenyl | Z | CH | |
| 76 | 3-Fluorphenyl | 2-Chlorphenyl | E | CH | |
| 77 | 3-Fluorphenyl | 4-Chlorphenyl | Z | N | |

| Nr. | $R^1$ | $R^2$ | Isomer | X | Schmp. |
|---|---|---|---|---|---|
| 78 | 3-Fluorphenyl | 4-Chlorphenyl | E | N | |
| 79 | 3-Fluorphenyl | 4-Chlorphenyl | Z | CH | |
| 80 | 3-Fluorphenyl | 4-Chlorphenyl | E | CH | |
| 81 | 3-Fluorphenyl | 2,4-Dichlorphenyl | Z | N | |
| 82 | 3-Fluorphenyl | 2,4-Dichlorphenyl | E | N | |
| 83 | 3-Fluorphenyl | 2,4-Dichlorphenyl | Z | CH | |
| 84 | 3-Fluorphenyl | 2,4-Dichlorphenyl | E | CH | |
| 85 | 3-Fluorphenyl | 2-Thienyl | Z | N | |
| 86 | 3-Fluorphenyl | 2-Thienyl | E | N | |
| 87 | 3-Fluorphenyl | 2-Thienyl | Z | CH | |
| 88 | 3-Fluorphenyl | 2-Thienyl | E | N | |
| 89 | 3-Fluorphenyl | 3-Thienyl | Z | N | |
| 90 | 3-Fluorphenyl | 3-Thienyl | E | N | |
| 91 | 3-Fluorphenyl | 3-Thienyl | Z | CH | |
| 92 | 3-Fluorphenyl | 3-Thienyl | E | CH | |
| 93 | 3-Fluorphenyl | 2-Furanyl | Z | N | |
| 94 | 3-Fluorphenyl | 2-Furanyl | E | N | |
| 95 | 3-Fluorphenyl | 2-Furanyl | Z | CH | |
| 96 | 3-Fluorphenyl | 2-Furanyl | E | CH | |
| 97 | 3-Fluorphenyl | 3-Furanyl | Z | N | |
| 98 | 3-Fluorphenyl | 3-Furanyl | E | N | |
| 99 | 3-Fluorphenyl | 3-Furanyl | Z | CH | |
| 100 | 3-Fluorphenyl | 3-Furanyl | E | CH | |
| 105 | 3-Fluorphenyl | 5-(3-Methylisoxazolyl) | Z | N | |
| 106 | 3-Fluorphenyl | 5-(3-Methylisoxazolyl) | E | N | |
| 107 | 3-Fluorphenyl | 5-(3-Methylisoxazolyl) | Z | CH | |
| 108 | 3-Fluorphenyl | 5-(3-Methylisoxazolyl) | E | CH | |
| 109 | 2,4-Difluorphenyl | 2-Chlorphenyl | Z | N | |
| 110 | 2,4-Difluorphenyl | 2-Chlorphenyl | E | N | |
| 111 | 2,4-Difluorphenyl | 2-Chlorphenyl | Z | CH | |
| 112 | 2,4-Difluorphenyl | 2-Chlorphenyl | E | CH | |
| 117 | 2,4-Difluorphenyl | 2,4-Dichlorphenyl | Z | N | |
| 118 | 2,4-Difluorphenyl | 2,4-Difluorphenyl | E | N | |
| 119 | 2,4-Difluorphenyl | 2,4-Dichlorphenyl | Z | CH | |

| Nr. | $R^1$ | $R^2$ | Isomer | X | Schmp. |
|---|---|---|---|---|---|
| 120 | 2,4-Difluorphenyl | 2,4-Dichlorphenyl | E | CH | |
| 121 | 2,4-Difluorphenyl | 2-Thienyl | Z | N | |
| 122 | 2,4-Difluorphenyl | 2-Thienyl | E | N | |
| 123 | 2,4-Difluorphenyl | 2-Thienyl | Z | CH | |
| 124 | 2,4-Difluorphenyl | 2-Thienyl | E | N | |
| 125 | 2,4-Difluorphenyl | 3-Thienyl | Z | N | |
| 126 | 2,4-Difluorphenyl | 3-Thienyl | E | N | |
| 127 | 2,4-Difluorphenyl | 3-Thienyl | Z | CH | |
| 128 | 2,4-Difluorphenyl | 3-Thienyl | E | CH | |
| 129 | 2,4-Difluorphenyl | 2-Furanyl | Z | N | |
| 130 | 2,4-Difluorphenyl | 2-Furanyl | E | N | |
| 131 | 2,4-Difluorphenyl | 2-Furanyl | Z | CH | |
| 132 | 2,4-Difluorphenyl | 2-Furanyl | E | CH | |
| 133 | 2,4-Difluorphenyl | 3-Furanyl | Z | N | |
| 134 | 2,4-Difluorphenyl | 3-Furanyl | E | N | |
| 135 | 2,4-Difluorphenyl | 3-Furanyl | Z | CH | |
| 136 | 2,4-Difluorphenyl | 3-Furanyl | E | CH | |
| 141 | 2,4-Difluorphenyl | 5-(3-Methylisoxazolyl) | Z | N | |
| 142 | 2,4-Difluorphenyl | 5-(3-Methylisoxazolyl) | E | N | |
| 143 | 2,4-Difluorphenyl | 5-(3-Methylisoxazolyl) | Z | CH | |
| 144 | 2,4-Difluorphenyl | 5-(3-Methylisoxazolyl) | E | CH | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse — wie Gurken, Bohnen und Kürbisgewächse —.

Die neuen Verbindungen sind insbesondere geeignet zur Bekämpfung folgender Pflanzenkrankheiten :

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach

der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser ; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin- Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind :

I. Man vermischt 90 Gew.-Teile der Verbindung Nr. 1 mit 10 Gew.- Teilen N-Methyl-α-pyrrolidon und erhält eine Lösung, die zur Anwendung in Form kleinster Tropfen geeignet ist.

II. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

III. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

IV. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

V. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäureharnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde 1-(1,2,4-Triazol-1-yl)-2-(2,4-dichlorphenyl)-pentan (A) — bekannt aus C.A. No. 66246-88-6 — benutzt.

Anwendungsbeispiel 1

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80% Wirkstoff und 20% Emulgiermittel in der Trockensubstanz enthielten, tropfnaß gespritzt. Nach dem Abtrocknen des Spritzbelages wurden die Versuchspflanzen im Gewächshaus bei Temperaturen zwischen 20 und 22°C und 65 bis 70% relativer Luftfeuchte aufgestellt. Nach 8 Tagen wurde das Ausmaß der Rostpilzentwicklung auf den Blättern ermittelt.

Das Ergebnis zeigt, daß der Wirkstoff 1 bei der Anwendung als 0,006%ige (Gew.%) Spritzbrühe eine bessere fungizide Wirkung zeigt (100%) als der bekannte Vergleichswirkstoff A (60%).

Anwendungsbeispiel 2

Wirksamkeit gegen Pyrenophora teres

Gerstenkeimlinge der Sorte "Igri" wurden im Zweiblattstadium mit wäßrigen Suspensionen, die 80% Wirkstoff und 20% Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20-22°C und 70% relativer Luftfeuchtigkeit für weitere 5 Tage kultiviert. Dann wurde das Ausmaß der Symptomentwicklung ermittelt.

Das Ergebnis zeigt, daß der Wirkstoff 1 bei der Anwendung als 0,025%ige Spritzbrühe eine sehr gute fungizide Wirkung (100%) zeigt.

Anwendungsbeispiel 3

Wirksamkeit gegen Pyricularia oryzae (protektiv)

Blätter von in Töpfen gewachsenen Reiskeimlingen der Sorte "Bahia" wurden mit wäßrigen Emulsionen, die 80% Wirkstoff und 20% Emulgiermittel in der Trockensubstanz enthielten, tropfnaß besprüht und 24 Stunden später mit einer wäßrigen Sporensuspension von Pyricularia oryzae inoculiert. Anschließend wurden die Versuchspflanzen in Klimakammern bei 22 bis 24°C und 95 bis 99% relativer Luftfeuchtigkeit aufgestellt. Nach 6 Tagen wurde das Ausmaß des Krankheitsbefalls ermittelt.

Das Ergebnis zeigt, daß der Wirkstoff 1 bei der Anwendung als 0,05%ige Spritzbrühe eine sehr gute fungizide Wirkung zeigt (97%).

## Patentansprüche

1. Azolylalkene der allgemeinen Formel I

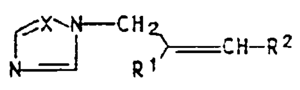

I

in welcher $R^1$ Fluorphenyl,
$R^2$ Naphthyl, einen 5- oder 6-gliedrigen Heterocyclus, Biphenyl oder Phenyl, wobei diese Reste gegebenenfalls durch Halogen, Nitro, Amino, Phenoxy, Alkyl, Alkoxy oder Halogenalkyl mit jeweils 1 bis 4 Kohlenstoffatomen substituiert sind, und
X CH oder N bedeuten, sowie deren für Pflanzen verträgliche Säureadditionssalze und Metallkomplexe, außer den Verbindungen, in denen $R^1$ 4-Fluorphenyl oder 2,4-Difluorphenyl und gleichzeitig $R^2$ 4-Fluorphenyl, 4-Chlorphenyl, 4-Methoxyphenyl, 4-Trifluormethylphenyl oder Pyridyl-4 bedeuten.

2. Verfahren zur Herstellung der Azolylalkene der Formel I

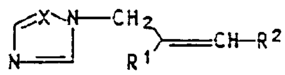

I

in welcher $R^1$ Fluorphenyl,
$R^2$ Naphthyl, einen 5- oder 6-gliedrigen Heterocyclus, Biphenyl oder Phenyl, wobei diese Reste gegebenenfalls durch Halogen, Nitro, Amino, Phenoxy, Alkyl, Alkoxy oder Halogenalkyl mit jeweils 1 bis 4 Kohlenstoffatomen substituiert sind, und
X CH oder N bedeuten, außer den Verbindungen, in denen $R^1$ 4-Fluorphenyl oder 2,4-Difluorphenyl und gleichzeitig $R^2$ 4-Fluorphenyl, 4-Chlorphenyl, 4-Methoxyphenyl, 4-Trifluormethylphenyl oder Pyridyl-4 bedeuten, dadurch gekennzeichnet, daß man eine Verbindung der Formel

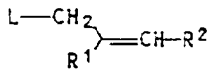

II

in welcher $R^1$, $R^2$ die oben angegebenen Bedeutungen haben und L eine nucleophil substituierbare Abgangsgruppe darstellt, mit einer Verbindung der Formel II

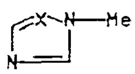

III,

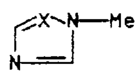

III,

in der Me ein Wasserstoffatom oder ein Metallatom bedeutet und X die oben angegebene Bedeutung hat, zur Umsetzung bringt, und die so erhaltenen Verbindungen gegebenenfalls in ihre Salze mit für Pflanzen verträglichen Säuren überführt.

3. Fungizides Mittel, enthaltend eine fungizid wirksame Menge eines Azolylalkens der allgemeinen Formel I

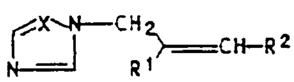

I

in welcher $R^1$ Fluorphenyl,
$R^2$ Naphthyl, einen 5-oder 6-gliedrigen Heterocyclus, Biphenyl oder Phenyl, wobei diese Reste gegebenenfalls durch Halogen, Nitro, Amino, Phenoxy, Alkyl, Alkoxy oder Halogenalkyl mit jeweils 1 bis 4 Kohlenstoffatomen substituiert sind, und
X CH oder N bedeuten, oder dessen für Pflanzen verträglichen Säureadditionssalzes oder Metallkomplexes, außer den Verbindungen, in denen $R^1$ 4-Fluorphenyl oder 2,4-Difluorphenyl und gleichzeitig $R^2$ 4-Fluorphenyl, 4-Chlorphenyl, 4-Methoxyphenyl, 4-Trifluormethylphenyl oder Pyridyl-4 bedeuten, und einen inerten Zusatzstoff.

4. Verfahren zur Bekämpfung von Pilzen, dadurch gekennzeichnet, daß man eine fungizid wirksame Menge eines Azolylalkens der allgemeinen Formel I

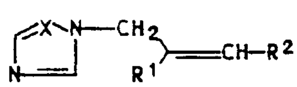

I

in welcher $R^1$ Fluorphenyl,
$R^2$ Naphthyl, einen 5- oder 6-gliedrigen Heterocyclus, Biphenyl oder Phenyl, wobei diese Reste gegebenenfalls durch Halogen, Nitro, Amino, Phenoxy, Alkyl, Alkoxy oder Halogenalkyl mit jeweils 1 bis 4 Kohlenstoffatomen substituiert sind, und
X CH oder N bedeuten, oder dessen für Pflanzen verträglichen Säureadditonssalzes oder Metallkomplexes, außer den Verbindungen, in denen $R^1$ 4-Fluorphenyl oder 2,4-Difluorphenyl und gleichzeitig $R^2$ 4-Fluorphenyl,

4-Chlorphenyl, 4-Methoxyphenyl, 4-Trifluormethylphenyl oder Pyridyl-4 bedeuten, auf die Pilze oder auf durch Pilzbefall bedrohte Materialien, Flächen, Pflanzen oder Saatgüter einwirken läßt.

5. Verbindung gemäß Anspruch 1, dadurch gekennzeichnet, daß $R^1$ 4-Fluorphenyl, X den Rest N und $R^2$ 2-Chlorphenyl bedeuten.

6. Verbindung gemäß Anspruch 1, dadurch gekennzeichnet, daß $R^1$ 4-Fluorphenyl, X den Rest N und $R^2$ 2-Thienyl bedeuten.

7. Verbindung gemäß Anspruch 1, dadurch gekennzeichnet, daß $R^1$ 4-Fluorphenyl, X den Rest N und $R^2$ 2-Furanyl bedeuten.

## Claims

1. An azolylalkene of the general formula I

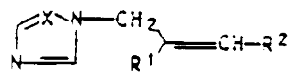
I

where $R^1$ is fluorophenyl, $R^2$ is naphthyl, a 5- or 6-membered heterocycle, biphenyl or phenyl, these radicals being unsubstituted or substituted by halogen, nitro, amino, phenoxy, alkyl, alkoxy or haloalkyl, each of 1 to 4 carbon atoms, and
X is CH or N, and its plant-tolerated acid addition salts and metal complexes, with the exception of the compounds in which $R^1$ is 4-fluorophenyl or 2,4-difluorophenyl and at the same time $R^2$ is 4-fluorophenyl, 4-chlorophenyl, 4-methoxyphenyl, 4-trifluoromethylphenyl or pyrid-4-yl.

2. A process for the preparation of an azolylalkene of the formula I

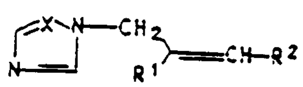
I

where $R^1$ is fluorophenyl, $R^2$ is naphthyl, a 5- or 6-membered heterocycle, biphenyl or phenyl, these radicals being unsubstituted or substituted by halogen, nitro, amino, phenoxy, alkyl, alkoxy or haloalkyl, each of 1 to 4 carbon atoms, and
X is CH or N, with the exception of the compounds in which $R^1$ is 4-fluorophenyl or 2,4-difluorophenyl and at the same time $R^2$ is 4-fluorophenyl, 4-chlorophenyl, 4-methoxyphenyl, 4-trifluoromethylphenyl or pyrid-4-yl, wherein a compound of the formula

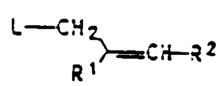
II

where $R^1$ and $R^2$ have the abovementioned meanings and L is a leaving group which can be nucleophilically substituted, is reacted with a compound of the formula III

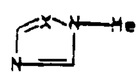
III

where Me is hydrogen or a metal atom and X has the abovementioned meaning, and the resulting compound is, if required, converted into its salts with plant-tolerated acids.

3. A fungicidal agent containing a fungicidally effective amount of an azolylalkene of the general formula I

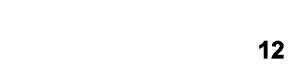
I

where $R^1$ is fluorophenyl, $R^2$ is naphthyl, a 5- or 6-membered heterocycle, biphenyl or phenyl, these radicals being unsubstituted or substituted by halogen, nitro, amino, phenoxy, alkyl, alkoxy or haloalkyl, each of 1 to 4 carbon atoms, and
X is CH or N, or a plant-tolerated acid addition salt or metal complex thereof, with the exception of the compounds in which $R^1$ is 4-fluorophenyl or 2,4-difluorophenyl and at the same time $R^2$ is 4-fluorophenyl, 4-chlorophenyl, 4-methoxyhenyl, 4-trifluoromethylphenyl or pyrid-4-yl, and an inert additive.

4. A process for controlling fungi, wherein a fungicidally effective amount of an azolylalkene of the general formula I

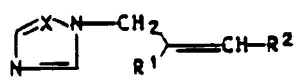

I

where $R^1$ is fluorophenyl, $R^2$ is naphthyl, a 5- or 6-membered heterocycle, biphenyl or phenyl, these radicals being unsubstituted or substituted by halogen, nitro, amino, phenoxy, alkyl, alkoxy or haloalkyl, each of 1 to 4 carbon atoms, and
X is CH or N, or a plant-tolerated acid addition salt or metal complex thereof, with the exception of the compounds in which $R^1$ is 4-fluorophenyl or 2,4-difluorophenyl and at the same time $R^2$ is 4-fluorophenyl, 4-chlorophenyl, 4-methoxyphenyl, 4-trifluoromethylphenyl or pyrid-4-yl, is allowed to act on the fungi or materials, areas, plants or seed threatened by fungus attack.

5. A compound as claimed in claim 1, wherein $R^1$ is 4-fluorophenyl, X is N and $R^2$ is 2-chlorophenyl.

6. A compound as claimed in claim 1, wherein $R^1$ is 4-fluorophenyl, X is N and $R^2$ is 2-thienyl.

7. A compound as claimed in claim 1, wherein $R^1$ is 4-fluorophenyl, X is N and $R^2$ is 2-furanyl.

## Revendications

1. Azolylalcènes de formule générale I

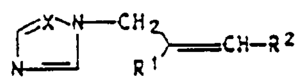

I

dans laquelle $R^1$ représente fluorophényle,
$R^2$, naphtyle, un hétérocycle à 5 ou 6 chaînons, biphényle ou phényle, ces restes étant éventuellement substitués par halogène, nitro, amino, phénoxy, alkyle, alcoxy ou halogène-alkyle ayant chacun 1 à 4 atomes de carbone, et
X, CH ou N, ainsi que leurs sels d'addition et complexes métalliques tolérés par les plantes, excepté les composés dans lesquels $R^1$ représente 4-fluorophényle ou 2,4-difluorophényle et $R^2$ en même temps 4-fluorophényle, 4-chlorophényle, 4-méthoxyphényle, 4-trifluorométhylphényle ou pyridyle-4.

2. Procédé de préparation des azolylalcènes de formule I

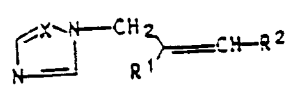

I

dans laquelle $R^1$ représente fluorophényle,
$R^2$, naphtyle, un hétérocycle à 5 ou 6 chaînons, biphényle ou phényle, ces restes étant éventuellement substitués par halogène, nitro, amino, phénoxy, alkyle, alcoxy ou halogène-alkyle ayant chacun 1 à 4 atomes de carbone, et
X, CH ou N, excepté les composés dans lesquels $R^1$ représente 4-fluorophényle ou 2,4-difluorophényle et $R^2$ en même temps fluorophényle, 4-chlorophényle, 4-méthoxyphényle, 4-trifluorométhylphényle ou pyridyle-4, caractérisé par le fait que l'on met à réagir un composé de formule

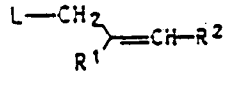

II

dans laquelle $R^1$ et $R^2$ ont les significations données plus haut et L représente un groupe de départ substituable par un réactif nucléophile, avec un composé de formule III

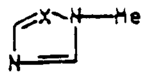

III.

dans laquelle Me représente un atome d'hydrogène ou un atome de métal, X ayant la signification donnée plus haut, et on transforme éventuellement les composés ainsi obtenus en leurs sels avec des acides tolérés par les plantes.

3. Agent fongicide contenant une quantité efficace du point de vue fongicide d'un azolylalcène de formule générale I

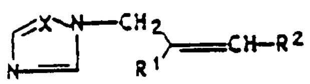

I

dans laquelle $R^1$ représente fluorophényle,
$R^2$, naphtyle, un hérérocycle à 5 ou 6 chaînons, biphényle ou phényle, ces restes étant éventuellement substitués par halogène, nitro, amino, phénoxy, allyle, alcoxy ou halogène-alkyle ayant chacun 1 à 4 atomes de carbone, et
X, CH ou N, ou de son sel d'addition d'acide ou complexe métallique tolérés par les plantes, excepté les composés dans lesquels $R^1$ représente 4-fluorophényle ou 2,4-difluorophényle et $R^2$ en même temps 4-fluorophényle, 4-chlorophényle, 4-méthoxyphényle, 4-trifluorométhylphényle ou pyridyle-4, et un produit d'addition inerte.

4. Procédé de lutte contre les champignons, caractérisé par le fait que l'on fait agir sur les champignons ou les matériaux, surfaces, plantes ou semences menacés par l'attaque par des champignons, une quantité efficace du point de vue fongicide d'un azolylalcène de formule générale I

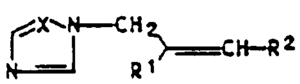

I

dans laquelle $R^1$ représente fluorophényle,
$R^2$, naphtyle, un hétérocycle à 5 ou 6 chaînons, biphényle ou phényle, ces restes étant éventuellement substitués par halogène, nitro, amino, phénoxy, alkyle, alcoxy ou halogène-alkyle ayant chacun 1 à 4 atomes de carbone, et
X, CH ou N, ou de son sel d'additon d'acide ou complexe métallique tolérés par les plantes, excepté les composés dans lesquels $R^1$ représente 4-fluorophényle ou 2,4-difluorophényle et $R^2$ en même temps 4-fluorophényle, 4-chlorophényle, 4-méthoxyphényle, 4-trifluorométhylphényle ou pyridyle-4.

5. Composé selon la revendication 1, caractérisé par le fait que $R^1$ représente 4-fluorophényle, X, le reste N et $R^2$, 2-chlorophényle.

6. Composé selon la revendication 1, caractérisé par le fait que $R^1$ représente 4-fluorophényle, X, le reste N et $R^2$, 2-thiényle.

7. Composé selon la revendication 1, caractérisé par le fait que $R^1$ représente 4-fluorophényle, X, le reste N et $R^2$, 2-furanyle.